# EUROPEAN PATENT SPECIFICATION

(11) **EP 2 023 793 B1**
(45) Date of publication and mention of the grant of the patent: **18.11.2015**
(21) Application number: 07794964.2
(22) Date of filing: 17.05.2007
(51) Int. Cl.: A61B 1/018, A61B 17/34

(54) **MULTI-INSTRUMENT SURGICAL ACCESS USING A SINGLE ACCESS PORT**
EINZELNER ZUGANG FÜR MEHRERE CHIRURGISCHE INSTRUMENTE
ACCÉS CHIRURGICAL DE MULTIPLES INSTRUMENTS UTILISANT UN UNIQUE ORIFICE D'ACCÉS

(30) Priority: 17.05.2006 US 801034 P; 17.05.2006 US 801113 P; 07.07.2006 US 819235 P; 24.04.2007 US 789381
(43) Date of publication of application: 18.02.2009
(73) Proprietor: TransEnterix, Inc., Morrisville, NC 27560 (US)
(72) Inventor: WILLIAMS, Michael, S., Santa Rosa, CA 95404 (US); STACK, Richard, S., Chapel Hill, NC 27514 (US); ORTH, Geoffrey, A., Sebastopol, CA 95472 (US); SMITH, Jeff, Petaluma, CA 94952 (US); GLENN, Richard, A., Chapel Hill, NC 27516 (US); FIFER, Daniel W., Windsor, CA 95492 (US); ATHAS, William L, NC 27517 (US)
(74) Representative: Millburn, Julie Elizabeth
(86) International application number: PCT/US2007/011795
(87) International publication number: WO 2007/136683

(56) References cited:
- EP-A- 1 586 275
- WO-A1-2005/009227
- WO-A1-2007/127199
- US-A- 5 318 013
- US-A1- 2004 138 525
- US-B1- 6 352 503

## Description

### FIELD OF THE INVENTION

The present invention relates to the field of devices for use in performing surgery in the peritoneal cavity using access through a single port in the abdominal wall.

### BACKGROUND OF THE INVENTION

Surgery in the abdominal cavity is typically performed using open surgical techniques or laparoscopic procedures. Each of these procedures requires incisions through the skin and underlying muscle and peritoneal tissue, and thus results in the potential for post-surgical scarring and/or hernias. Laparoscopic procedures, while less invasive than open surgical techniques, require multiple small incisions or ports to gain access to the peritoneal site using the various instruments and scopes needed to complete the procedure. The systems disclosed herein allow such procedures to be performed using only a single port.

US 5,318,013 discloses a surgical device including a frame member having a pair of clamping mechanisms movably mounted to the frame member at the distal end thereof for exerting respective clamping forces on spaced organic tissues of a patient. Actuators mounted to the frame member at the proximal end thereof are operatively connected to the clamping mechanisms for controlling the operation thereof. Another actuator, also mounted to the frame member, is operatively connected to the clamping mechanisms for increasing a distance therebetween, thereby stretching organic tissues between the clamped tissues and facilitating access to the stretched tissues.

### BRIEF DESCRIPTION OF THE DRAWINGS

Fig. 1A is a perspective view showing a first embodiment of a single port surgical system.
Fig. 1B is cross-section view taken along the plane designated 1B-1B in Fig. 1A.
Fig. 2A is a top perspective view showing the distal portion of the single port surgical system of Fig. 1A.
Figs. 2B and 2C are a top plan view and a side elevation view of the linkage assembly of Fig. 2A. In Fig. 2C, the center retractor is shown in a downwardly deflected position, and phantom lines are shown to illustrate the retractor in an upwardly deflected position.
Fig. 2D is a top plan view of the linkage assembly of Fig. 2A in the streamlined position.
Fig. 2E is a perspective view similar to Fig. 2A illustrating exemplary movement patterns for the tool cannulas and associated tools.
Fig. 3A is a perspective view showing the distal end of slightly modified single port surgical system using an alternative linkage configuration.
Fig. 3B is a cross-section view taken along the plane designated 3B-3B in Fig. 3A.
Figs. 4A and 4B are a top perspective view and a bottom perspective view, respectively, of the distal end of another embodiment using an additional tool cannula.
Figs. 5 and 6 are a perspective view and a cross-sectional side view of a gimbal assembly.
Figs. 7A and 7B are perspective views of the gimbal assembly of Fig. 5 showing two exemplary locking mechanisms.
Figs. 8A and 8B are perspective views of an alternative gimbal system.
Fig. 9 is a detailed perspective view of the proximal end of a procedural cannula and support system using yet another alternative gimbal system.
Fig. 10 shows the gimbal system of the Fig. 9 embodiment.
Fig. 11 is an exploded view of the gimbal system of Fig. 19.
Fig. 12 is a plan view of the distal surface of the ball of the gimbal system of Fig. 10.
Fig. 13 is a plan view of the proximal surface of the ball of Fig. 12, with the cap removed and shown in perspective view.
Fig. 14 is a perspective view of an alternative user interface for the system of Fig. 1A.
Fig. 15 is a perspective view showing an alternate single port surgical system having a detachable proximal component. The proximal and distal components are show separated from one another.
Fig. 16 is a detailed view of a portion of the system of Fig. 15 showing the socket and the hub. The socket is shown partially cut-away to permit viewing of features located inside it.
Figs. 17A and 17B are perspective views of a pullwire head and control wire connector illustrating the step of coupling the two together.
Figs. 18A and 18B are perspective views of one embodiment of an access cannula.
Fig. 19A is a perspective view of a second embodiment of an access cannula.
Fig. 19B is a side elevation view of a modification to the embodiment of Fig. 19A.
Fig. 20 schematically illustrates the single port surgical system of Fig. 1A coupled to a surgical table and having its distal end extending through an access cannula and into an insufflated abdominal cavity.
Fig. 21 schematically illustrates the single port surgical system of Fig. 1A coupled to a ceiling mount in a surgical theatre and having its distal end extending through an access cannula and into an insufflated abdominal cavity.
Fig. 22 schematically shows a patient lying prone on a surgical table and illustrates the system of Fig. 1A in use for surgery on a liver. The patient is shown as partially transparent to allow the system to be seen.

### DETAILED DESCRIPTION OF THE DRAWINGS

### Procedural Cannula and Support System

The system illustrated in the accompanying drawings allows surgical procedures to be carried out through a single port formed in an abdominal wall. The port may be formed using conventional techniques in a chosen location, or it may be formed through the umbilicus.

For certain procedures, it would be advantageous to allow the surgeon to perform a single port surgical procedure in a manner that allows him/her to approach the surgical target within the peritoneal cavity from the same direction from which s/he would typically approach that same structure using a multi-port laparoscopic or open surgical procedure. For example, if a particular procedure utilizes an anterior approach to the treatment site when carried out using laparoscopic or surgical techniques, it would also be desirable to allow the surgeon to approach the treatment site from an anterior perspective even when using a single port technique. It is also desirable to orient the tools in a single port system so they will approach the operative tissue site in the abdominal cavity from the same direction from which those same tools would have approached the site if introduced through separate ports using known laparoscopic techniques. The system illustrated in the attached figures allows familiar laparoscopic approaches to be used using single port access, thus allowing a surgeon to easily and intuitively transition between single port surgical procedures and multi-port laparoscopic procedures.

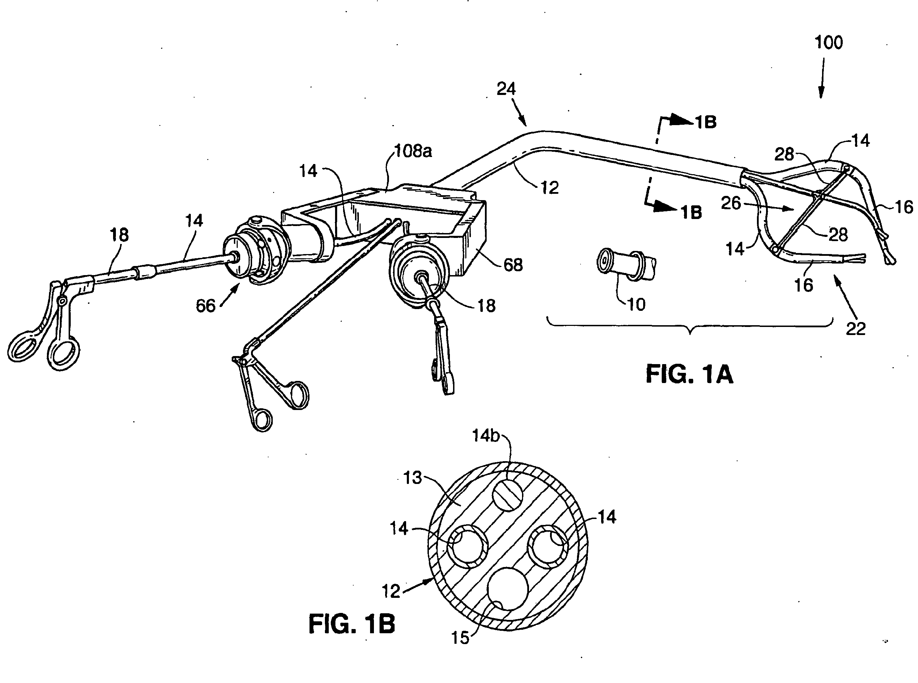

Referring to Fig. 1, one embodiment of a single port surgical system 100 includes an instrument system 22 and a support system 24. In use, the support system 24 forms a sort of scaffold or chandelier within the body to support the instrument system 22 in a location that allows the surgeon to advance the instruments of the instrument system using a desired approach. Thus, for example, if performing a procedure that typically uses an anterior approach when carried out surgically or laparoscopically, the user might position the support system 24 adjacent the interior of the abdominal wall.

Support system 24 includes an elongate overtube 12 that is extendable through an opening in a body wall, and preferably through an access cannula 10 positioned in an incision or trocar puncture in the abdominal wall. The overtube 12 is a rigid or semi-rigid tubular cannula, although it may be deployable in a more flexible state and later converted to a self-supporting rigid state similar to the locking spine described in U.S. Patent Application Publication No. 2008/0045803 A1, Filed April 24, 2007.

Referring again to Fig. 1A, instrument system 22 includes one or more procedural cannulas or tool cannulas 14 each having a lumen extending its length. Instruments 16 (e.g., forceps, endoscopes, suture devices, staplers) are extendable through the procedural cannulas 14 and into position at the target site in the peritoneal cavity, with the handles 18 of the instruments remaining outside the body. Two or three procedural cannulas are useful in that they allow for the simultaneous use of multiple instruments 16. In the Fig. 1A embodiment, a central retractor 14b is positioned between the tool cannulas 14. Retractor 14b has a handle 18b that can be manipulated to open/close the retractor jaws.

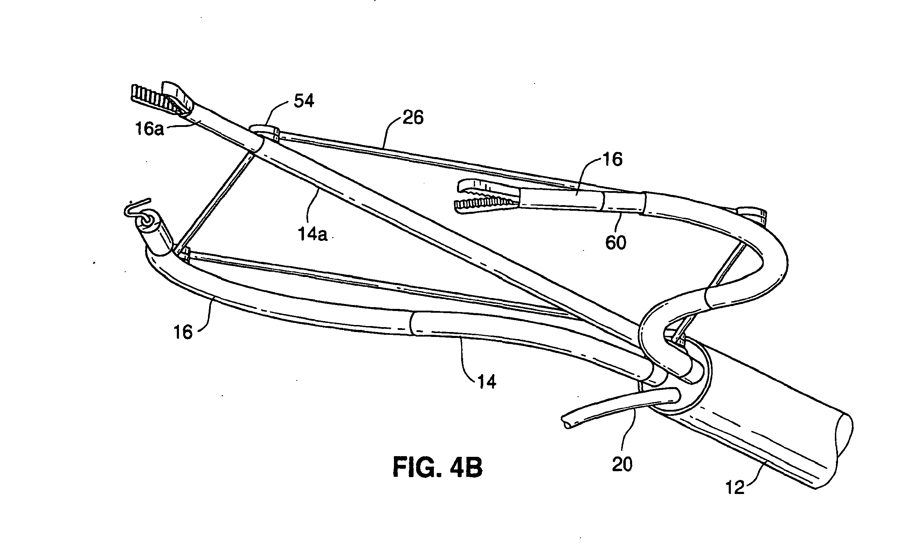

The procedural cannulas 14 and central retractor 14b extend through the overtube 12, allowing for a streamlined system that occupies a minimal amount of space. An endoscope 20 (Fig. 4B) can also extend through the overtube 12, allowing the user to observe the procedure being carried out at the distal end of the system. If needed, other instruments may extend directly through the overtube 12 towards the operative site and/or they may be supported by additional procedural cannulas.

If the system is to be used in procedures requiring insufflation, all or a portion of the length of the overtube may be filled with a plug formed of fill material 13 such as silicone or UV-curable polymer as shown in Fig. 1B. The fill material forms a seal around the procedural cannulas to prevent leakage of insufflation gas through the overtube. An additional endoscope lumen 15 may extend through the fill material for receiving an endoscope. The inner features of central retractor 14b are not shown in Fig. 1B.

Although the overtube 12 is described as formed of tubing, it can be replaced by any other structure that will bundle the tool cannulas and associated devices or cannulas (e.g. an endoscope or a cannula for the endoscope). As one example, instead of extending the tool cannulas etc. through an overtube, these devices may instead be bound together using shrink wrap or similar processes.

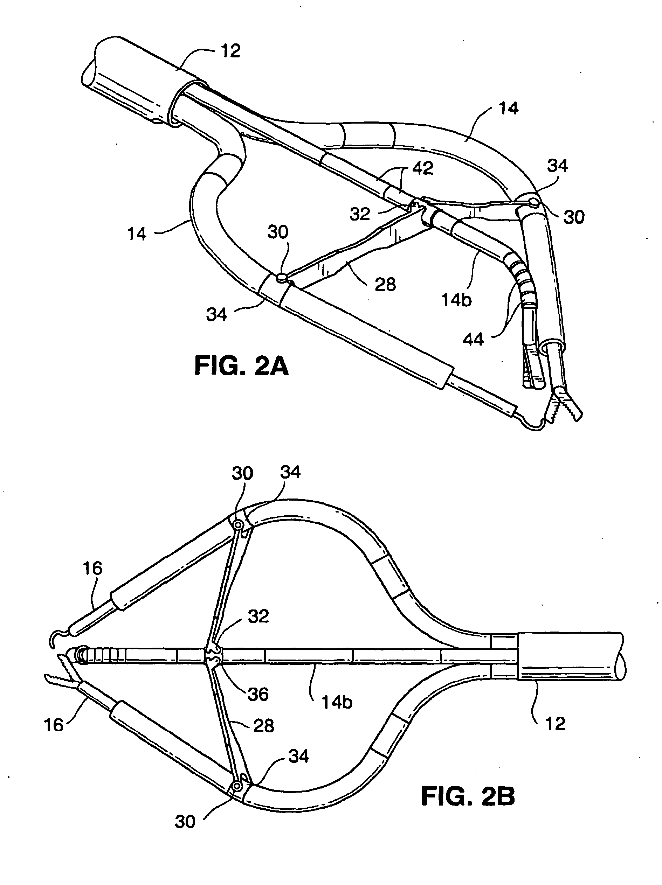

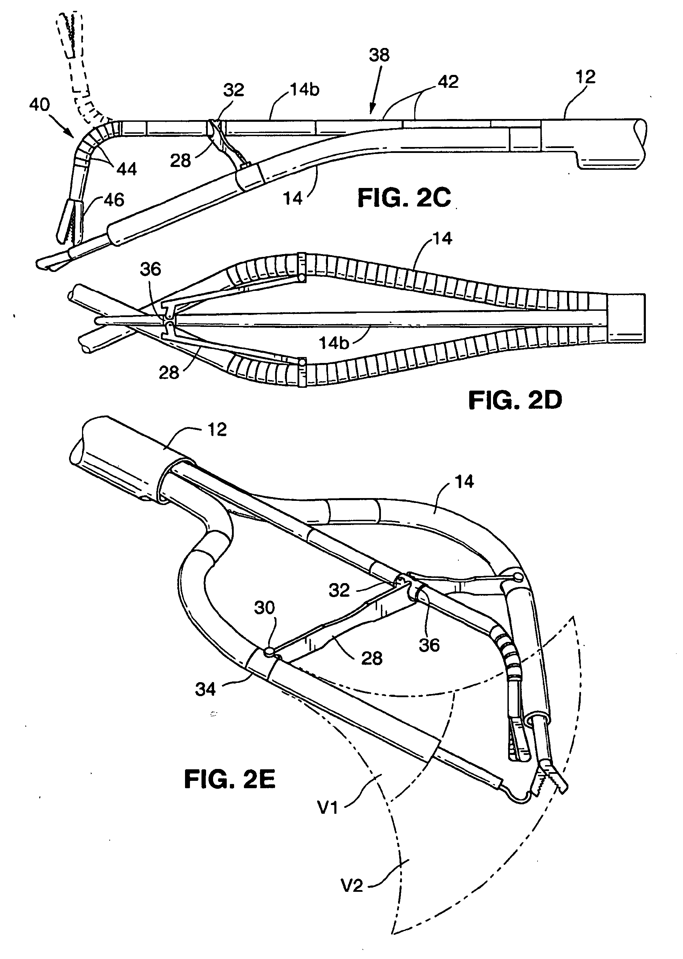

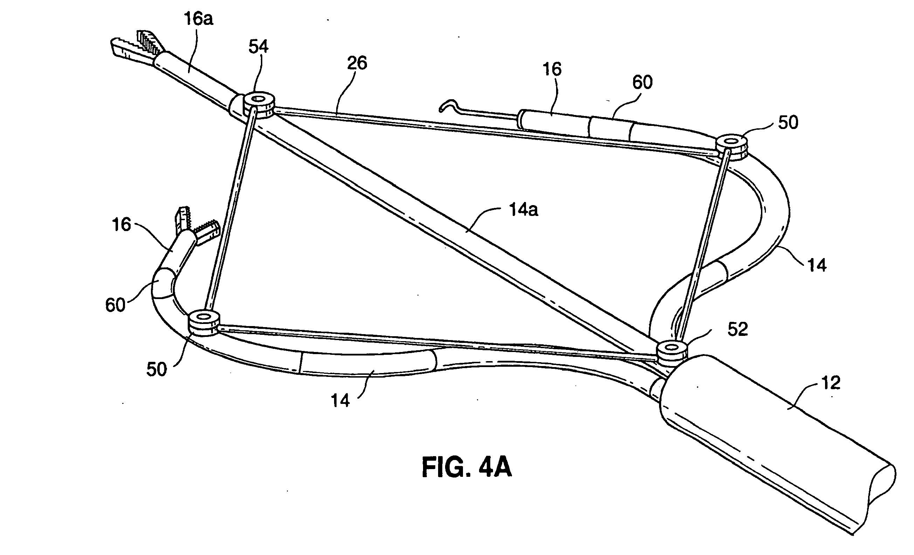

The system 100 includes features that support and orient the procedural cannulas 14 as appropriate for a given procedure. Referring to Fig. 1A, the tool cannulas are supported by a linkage system 26. In this embodiment, the linkage system 26 includes a pair of members 28. Each member 28 is attached by a corresponding one of the tool cannulas 14 by a first hinge 30 and to central retractor 14b (or, alternatively, to a longitudinal tool cannula like cannula 14a of Fig. 4A) by a second hinge 32. Hinges 30 may be mounted to corresponding collars 34 on the tool cannulas 14, and hinge 32 may be on a similar collar 36 (Fig. 2B) on retractor 14b. When linkage 26 is in the collapsed streamlined position, members 28 extend in a distal direction as shown in Fig. 2D, with the tool cannulas 14 disposed near the longitudinal axis of the overtube for passage through the access cannula 10. To deploy the linkage 26, central retractor 14b is withdrawn proximally, causing the members 28 to pivot at hinges 30, 32.

Referring to Fig. 2C, central retractor 14b includes a proximal section 38 and a distal section 40. Proximal section 38 is formed of a number of segments 42 strung onto one or more cables, with shorter segments 44 and an instrument tip 46 on the distal section 40. Cables within the retractor 14b are arranged such that the retractor becomes rigid when the cables are tensioned, and such that distal section 40 will deflect when the balance of tension within the cables is altered using controls (not shown) on the handle 18b or elsewhere outside the body. For example, retractor 14b may be deflectable towards and away from the body tissue as shown in Fig. 2C to allow tissue to be lifted by the retractor so the tissue may be acted upon by an instrument carried by one of the tool cannulas 14. Additional pull cables (not shown) are operable to open and close the jaws of the retractor tip 46.

In the disclosed embodiments, each tool cannula 14 preferably has a pre-shaped curve in its distal region. The curve orients the cannula 14 such that when the linkage is opened, the instruments 16 (Fig. 1 A) passed through the central lumens of the cannulas 14 can access a common treatment site. The preformed shape may be set using any of a number of methods. For example, cannulas 14 can be made of pre-curved tubing having rigidity sufficient to prevent buckling during use. Reinforcing braid made of stainless steel or other materials may be formed into the walls of the tubing in the rigid section of the cannulas 14. In other embodiments, the shaped region may have a segmented construction as shown in Fig. 2D (in which the linkage is in the collapsed position) and as similar to the segmented spine disclosed in U.S. Patent Application Publication No. 2008/0045803 A1, Filed April 24, 2007. With this design, individual spine segments are strung over one or more cables. The segments have individual shapes that collectively will give the tool cannulas the desired curvature (e.g. one that orients the cannulas as shown in Fig. 2A) when the cables running through the segments are tensioned. The entire length of the cannula may be segmented, or the distal portion may be formed of polymer tubing to allow flexibility.

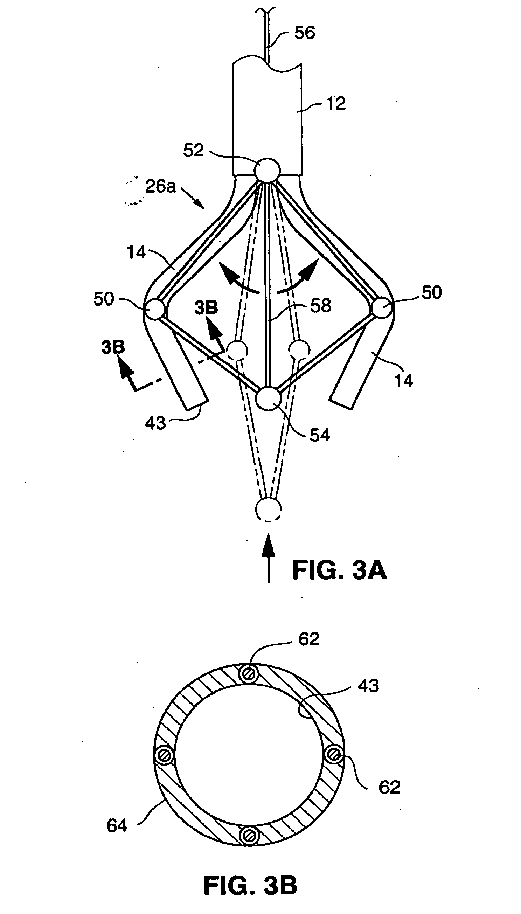

Fig. 3 A is a perspective view of modified configuration for the distal end of the system 100, showing the distal ends of the tool cannulas 14. In this embodiment, a linkage 26a is pivotally connected to the cannulas 14 at pivot points 50 and couples the cannulas 14 to the overtube 12. Linkage 26a also provides structural support for the distal portions of the tool cannulas 14 and maintains the relative orientation of the cannulas 14.

The linkage 26a is attached to a pivot mount 52 on the distal portion of the overtube 12. Another of the pivot mounts 54 is coupled to a pull wire 56 that extends proximally through overtube 12 to a location outside the body. In an alternative embodiment shown in Figs. 4A and 4B, pivot mount 54 may be coupled to the distal portion of a third longitudinal tool cannula 14a extending longitudinally from the overtube 12, or to a similarly positioned tool shaft (e.g. shaft 14b, Fig. 2A). As another alternative, either or both of the pivot mounts 52, 54 may extend into free space as shown instead of being attached to the cannula 14a and/or overtube 12.

Dashed lines in Fig. 3 A show the arrangement of the linkage 26a and pivot mounts 50 when that embodiments in the collapsed position. When in the streamlined position, the pivot mounts 50 are positioned side by side, thus orienting the tool cannulas 14 adjacent to one another. When in the deployed position, the pivot mounts are positioned approximately 7.5 - 18 cm (approximately 3 - 7 inches) apart, and more preferably approximately 10 cm -15 cm (approximately 4-6 inches) apart. In other words, the lateral separation between the tool cannulas within the body (i.e. in a direction perpendicular to the longitudinal axis of the overtube 12) may be in the range of 7.5 - 18 cm (3 - 7 inches).

The linkage 26a of Fig. 3A may be deployed to the open position by withdrawing pullwire 56, whereas the Fig. 4A, 4B embodiment can be deployed by advancing the distal end of the longitudinal tool cannula 14c in a distal direction to move the linkage 26a out of the access cannula and/or to deploy the linkage to the expanded position. In other embodiments, one or more of the pivot points 50, 52, 54 may be spring loaded to facilitate expansion of the linkage 26a. Any combination of these deployment mechanisms, or others not specifically mentioned, may instead be used to deploy the linkage 26a in the peritoneal cavity.

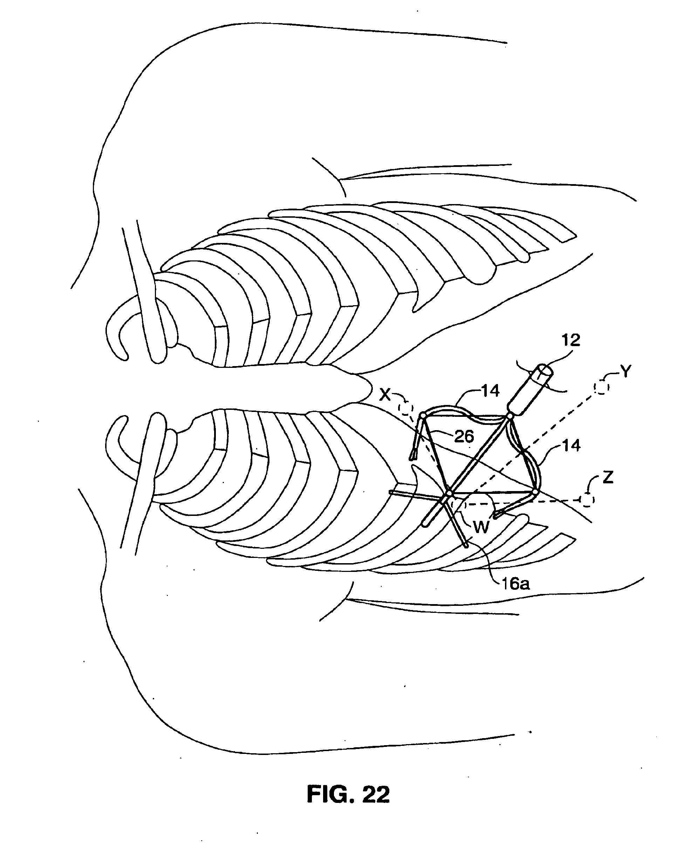

Opening the linkage positions the cannulas 14 as shown in Figs. 2A, 3A and 4A-4B and thus points the instruments 16 positioned in the cannulas 14 generally towards an operative tissue site. Once deployed within the body, a preferred system orients the tool cannulas 14 such that the tools 16 within the cannulas approach the tissue site from angles mimicking the angles of approach that those tool would have if introduced using a multiport laparoscopic procedure. This concept is discussed in greater detail in connection with Fig. 22.

The distal end of each tool cannula 14 has a region that is deflectable in multiple directions to allow positioning and manipulation of the operative ends of the instruments. This avoids the need for sophisticated steerable surgical instruments. Instead, instruments 16 having flexible shafts are positioned in the tool cannulas 14, and steering of the instruments is achieved by deflecting the tool cannulas 14. Because the tools 16 are flexible, it may be necessary to "stiffen" the shaft of the tool 16 to allow the tool to be successfully used. A slideable stiffening cannula 60 (Fig. 4A) may be advanced from within the tool cannula 14 over a portion of the shaft of the tool 16 to effectively stiffen the tool's shaft during the procedure, thus allowing the tool to be pressed into contact with body tissue without buckling. Other internal structures such as stiffening mandrels, reinforcing collars or braids, may instead be used for this purpose. The segmented or "shape-lock" construction described above in connection with Fig. 2D may also be used for the tool cannulas to provide rigidity to the cannulas during tool usage.

In a preferred embodiment, deflection of the tool cannulas 14 is performed using a pullwire system. Referring to Fig. 3B, pullwires 128 extend through corresponding pullwire lumens 64, preferably spaced at intervals of 90°. The distal ends of the pullwires are anchored in the distal sections of the cannula 14 such that the distal section of the cannula can be made to deflect in a desired direction by pulling on the desired combination of pullwires. Fig. 2E illustrates in dashed lines V1 a conical volumes defined by an exemplary movement pattern for the tool cannula 14, and the corresponding volume V2 defined by the tool 16 within the cannula 14.

Actuation of the pullwires is achieved using features that during use are positioned outside the body. A deflection system is provided that allows the user to intuitively actuate the pullwires for a particular one of the tool cannulas 14 by manipulating the handle 18 of the instrument 16 that resides within that tool cannula. For example, if the user wishes to have the distal end of a tool move in a downward direction, s/he will intuitively raise the handle 18 of that tool to cause the corresponding tool cannula to deflect downwardly, thus moving the tool to the desired position.

Referring to Fig. 1A, the proximal ends of the pullwires 62 extend from the proximal ends of the cannulas 14 and feed into a corresponding deflection system, which in the illustrated embodiments is a control gimbal 66.

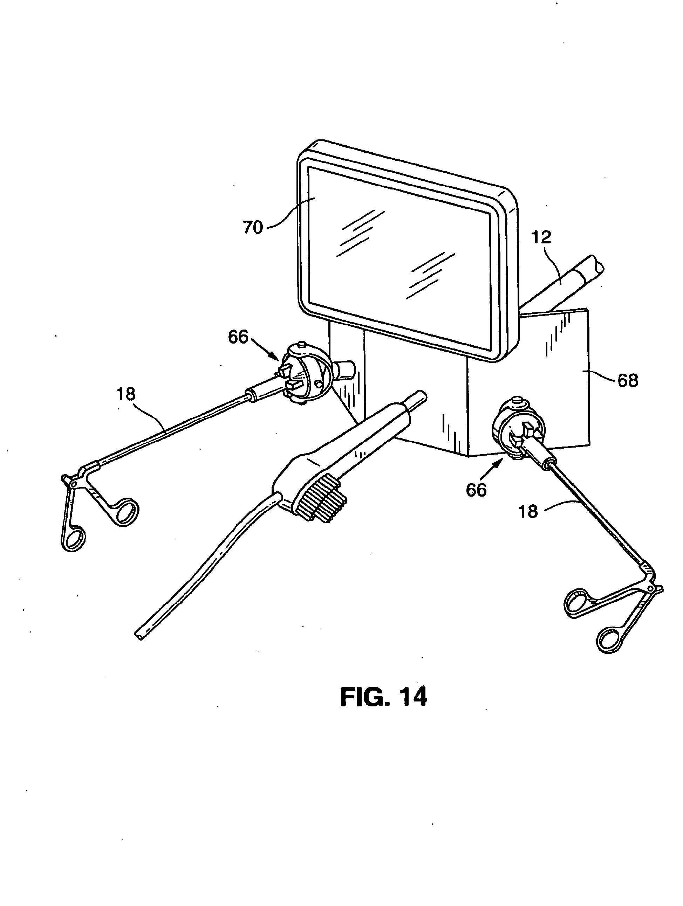

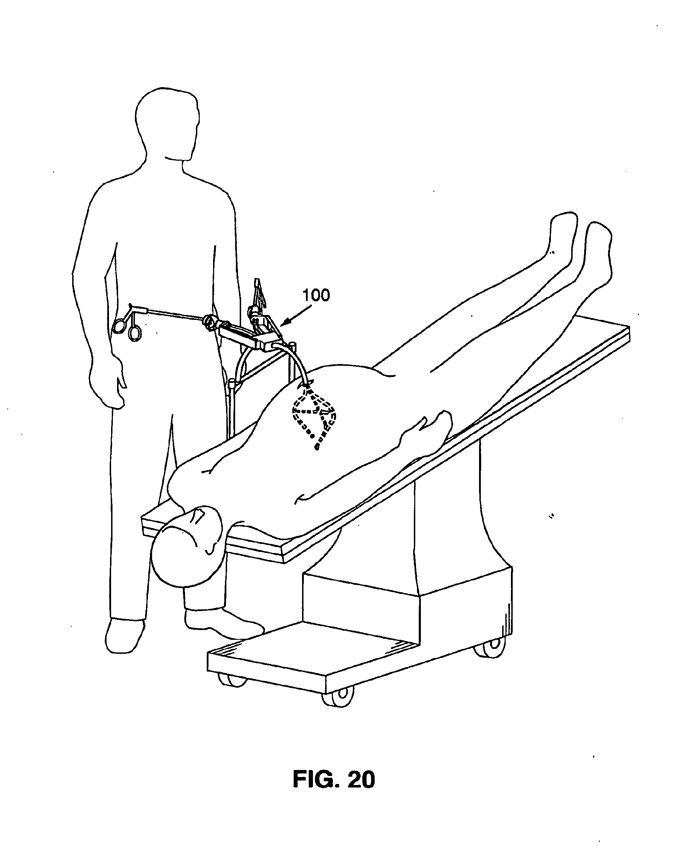

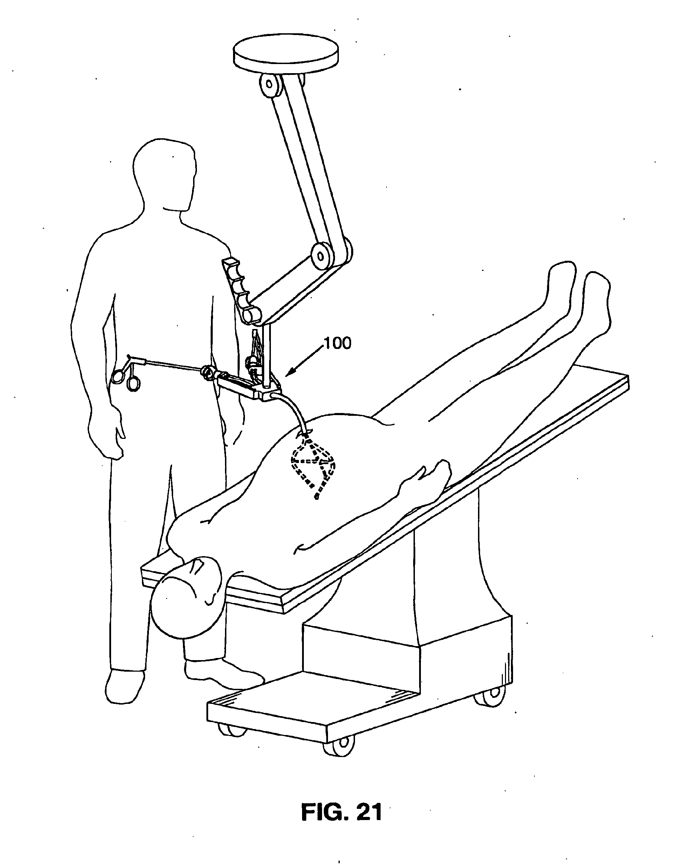

The gimbal 66 may be mounted to a work stand 68 as shown in Fig. 1A. In use the work stand 68 may be set on top of the patient's torso, mounted to a fixture within the operating room. The fixture might be one or both side-rails of the surgical table (Fig. 20), the ceiling of the surgical theatre (Fig. 21) or a cart positioned near the surgical table. In any case, the work stand 68 is positioned to give the surgeon convenient and intuitive access to the handles 18 while s/he observes the procedure on an endoscopic display (not shown). As shown in Fig. 14, use of the system may be facilitated by providing a "cockpit" for the user, coupling an endoscopic display 70 to a work stand 68 that supports the control gimbals 66, as well as the proximal controls for the endoscope 20, and optionally other ports for passing instruments through the access cannula to the peritoneal space.

The work stand 68 is proportioned to allow the surgeon to position his or herself in a comfortable position with his/her hands on the handles 18 of the tools 16. The work stand 68 preferably positions the tool handles 18 approximately 10-15 inches apart.

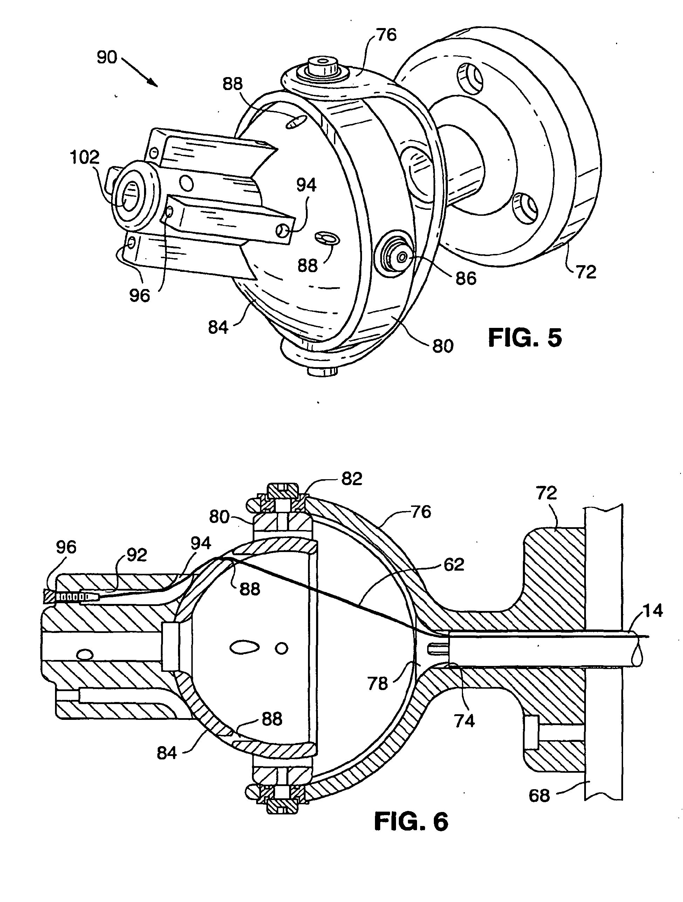

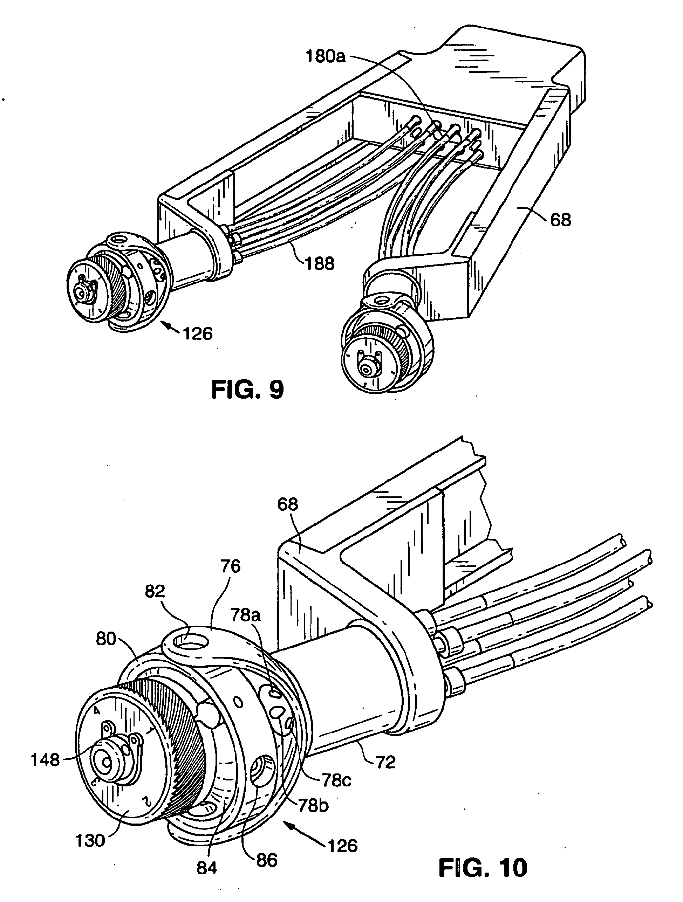

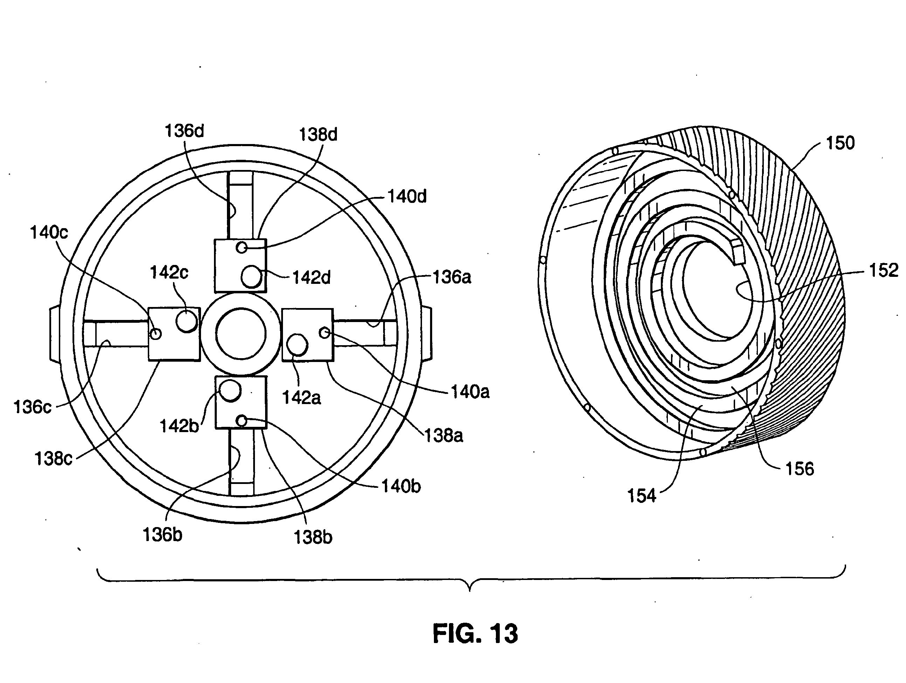

A preferred control gimbal 66 is shown in Fig. 13. It includes a base 72 mounted to the work stand (not shown in Fig. 5) and having a tubular end piece having a channel 74. A c-shaped mount 76 is connected to the base 72 and includes a through hole 78 continuous with the channel of the tubular end piece 74. In a slight modification, the hole 78 might be accompanied by four separate through holes 78a-d might be used for receiving pull wires as in the Fig. 10 embodiment to be discussed below. A ring 80 is pivotally mounted to the mount 76 at pivot bearings 82. A semi-spherical ball 84 is pivotally mounted within the ring at pivots 86. Four pull-wire ports 88 extend from the interior of the ball 84 to its outer surface.

Instrument port 90 includes side channels 92 having distal openings 94 and proximal openings 96. The four pullwires 62 from the tool cannulas 14 extend through the tubular end piece 74 and each passes through hole 78, through the hollow interior of the ball 84, and out corresponding ones of the pull-wire ports 88 in the ball. The pullwires further extend into the instrument port side channels 92 and are secured there by anchors 98.

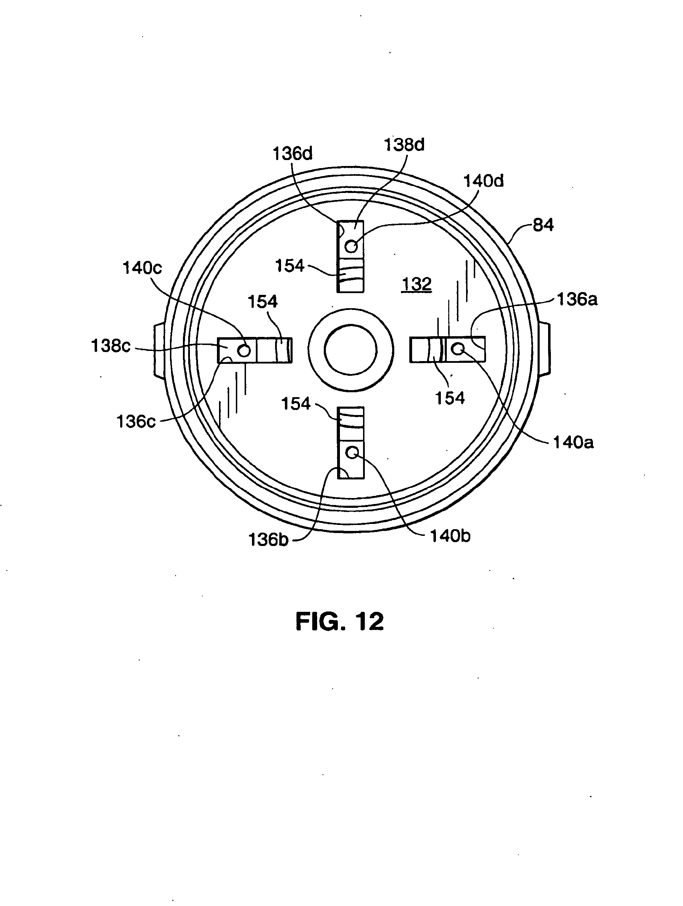

Instrument port 90 has a lumen 102 extending proximally from the spherical ball 84. The shaft 18 of an instrument 16 (see Fig. 12A, not shown in Figs. 13-14) extends through the lumen 102 and the ball 84, through hole 78 in the c-shaped mount 76, and via tube 74 and the work stand 68 (Fig. 12A), into the corresponding tool cannula 14. The operative end of the instrument 16 extends from the distal end of the tool cannula 14.

When it becomes necessary for the surgeon to change the orientation of the distal end of an instrument 16, s/he need only intuitively move the handle 18 of that instrument and the distal portion of the instrument will deflect accordingly as a result of the action of the gimbal on the pullwires of the tool cannula. Vertical movement of the handle 18 will cause the ball 84 to rotate relative to pivots 86, thus applying tension to the upper or lower pullwire 62 to cause upward or downward deflection of the tool cannula 14 (and thus the distal end of the instrument 16). Lateral movement of the handle 18 will cause the ball 84 and ring 80 to rotate about pivots 82 and to therefore tension one of the side pullwires to change the lateral bend of the tool cannula 14. The control gimbal allows combinations of vertical and lateral deflection, giving 360° deflection as shown in Fig. 4E. Thus user may additionally advance/retract the tool 16 longitudinally within the tool cannula 14, and/or axially rotate the tool 16 relative to the tool cannula when required.

The control gimbal 66 includes a locking mechanism that allows an instrument orientation to be temporarily fixed until further deflection is needed. This feature allows a user to fix a trajectory for multiple instruments that are to be sequentially used at a particular location. For example, once the orientation of a tool cannula 14 is set, a certain step in the procedure may be performed using a first instrument passed through that cannula. When a subsequent step requiring a different instrument is to be performed, the instruments are exchanged without moving the tool cannula 14. This allows the second instrument to be advanced to the exact location at which it is needed without additional steering.

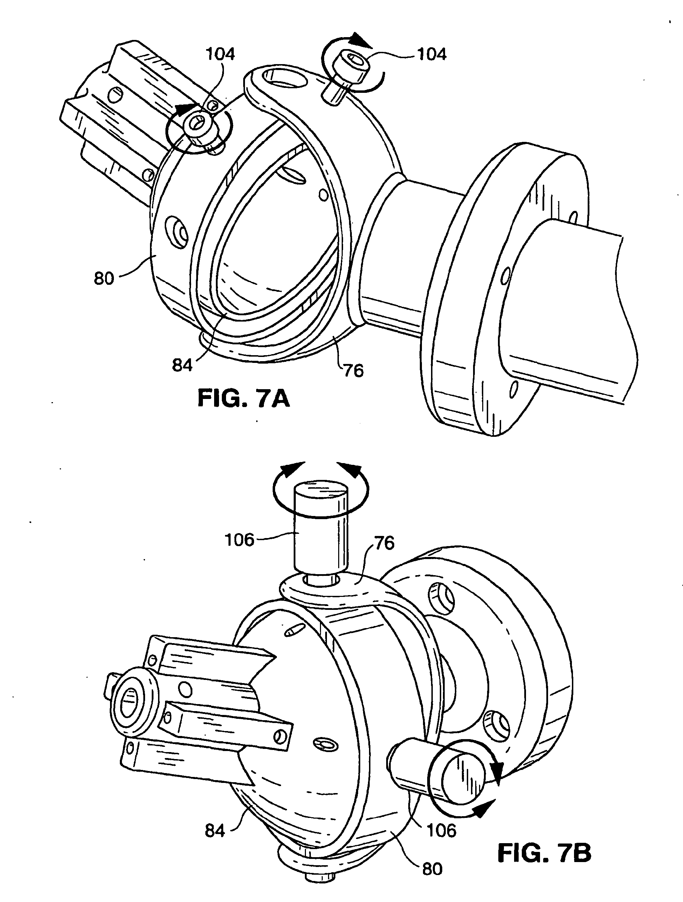

One exemplary locking mechanism includes a pair of locking screws 104 that are tightened as shown by arrows in Fig. 7A to lock the C-mount 76 to the ring 80 and to lock the ring 80 and the ball 84. Alternatively, as shown in Fig. 7B, a simple pneumatic shaft lock 106 could be employed on each of the gimbals' pivot axes. A solenoid or similar device might be used in place of the pneumatic lock 106.

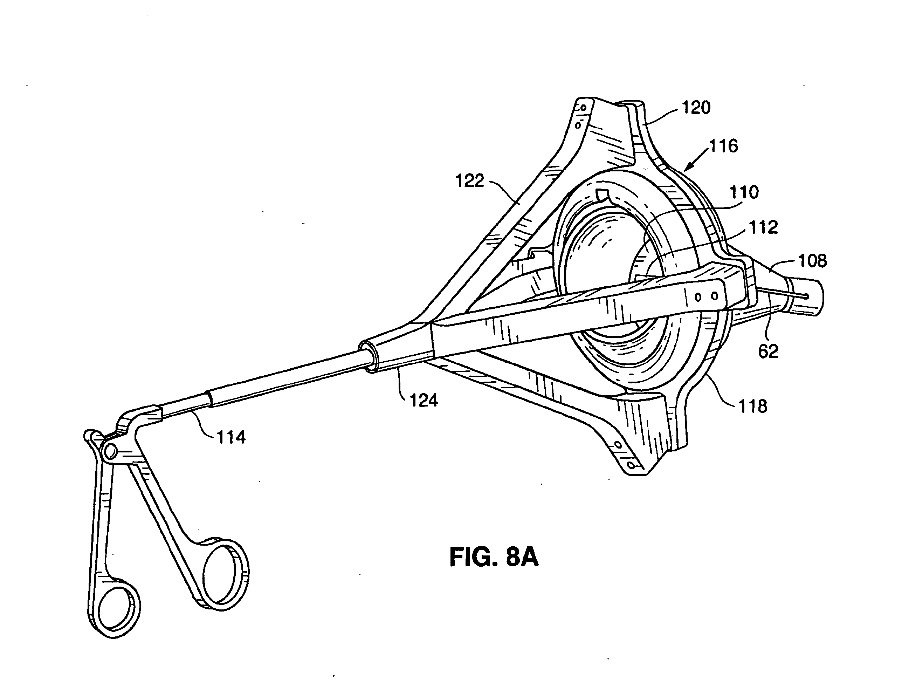

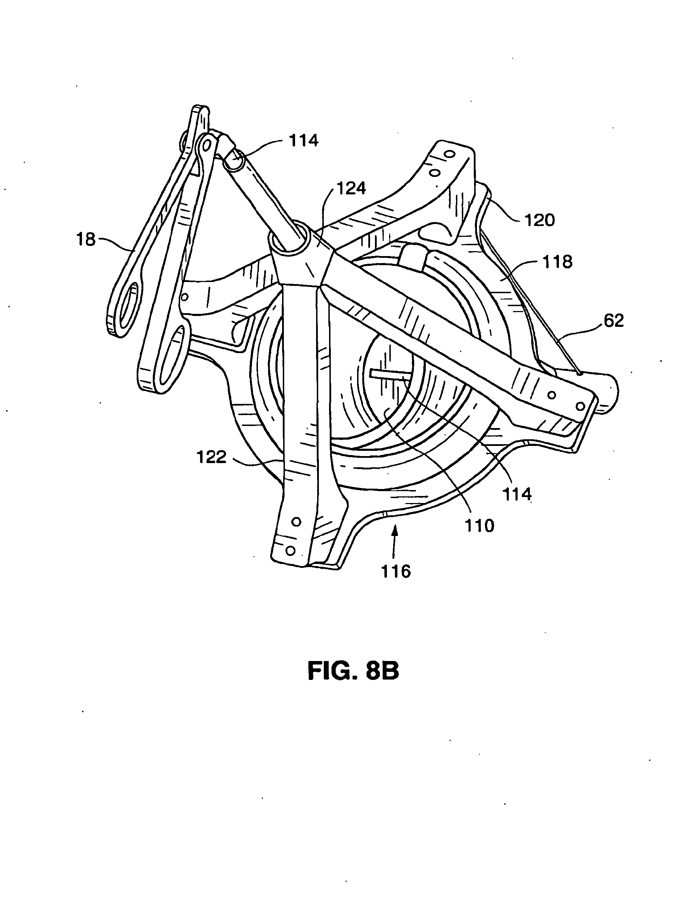

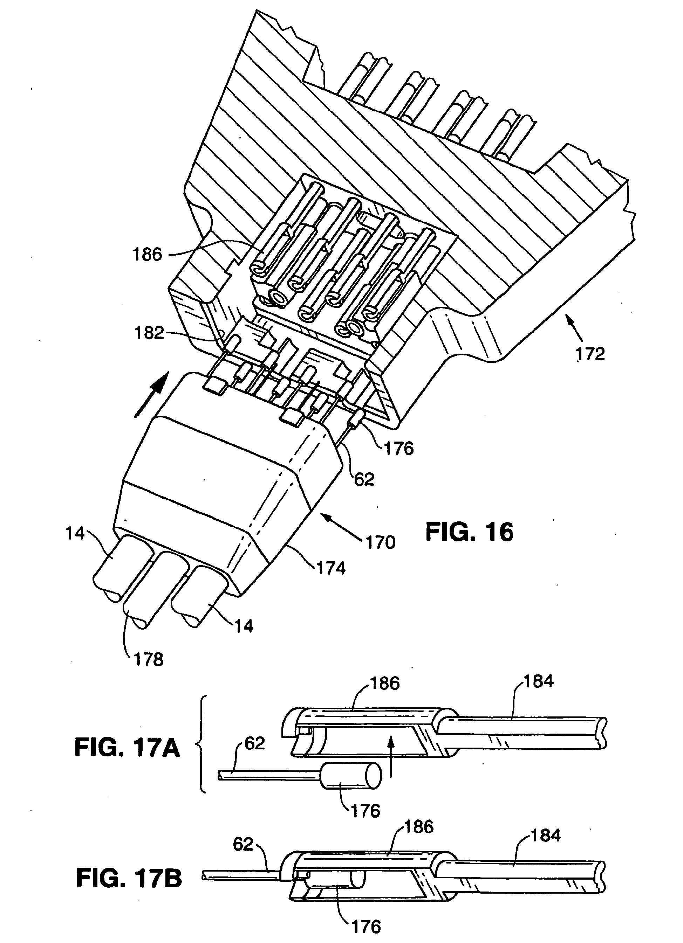

An alternate gimbal arrangement is shown in Figs.8A and 8B. As shown, a cone shaped instrument port 108 is mounted to the proximal end of each cannula, and includes a diaphragm seal 110 having a slit 112 sealable around an instrument shaft 114 passed into the instrument port 108. In Figs. 16A and 16B only the handle of instrument shaft 114 is shown to permit easier viewing of the surrounding features.

A gimbal 116 includes a collar 118 mounted on the instrument port 108 and four wings 120 radiating from the collar 118. Each pullwire 62 is coupled to one of the wings 120. Struts 122 extend proximally from the wings 120 and are joined to a sleeve 124 through which a portion of the instrument shaft 114 extends. Collar 118 is moveable relative to the instrument port 108, and in particular collar 118 is rotatable about its central axis, and pivotable in multiple directions. Movement of the collar 118 places one or more of the pullwires 62 under tension and results in deflection of the cannula 14. Since the instrument shaft 114 is coupled to the collar 118 by struts 122, a user can manipulate the instrument shaft 114 handle in an intuitive manner similar to a joystick to allow the user to steer the distal end of the cannula 14 in the desired direction.

Figs. 9-10 illustrate a gimbal system similar to that described in connection with Fig. 5, but that is modified to allow a user to adjust the sensitivity of the gimbals. In other words, the gimbal can be fine tuned such that the amount of deflection of the tool cannulas corresponds directly to the amount by which the user moves the tool handles 18 within the gimbal system, or the amount of deflection can be greater than or less than the corresponding movement of the tool handles.

Referring to Fig. 10, many of the features of the gimbal 126 are similar to those of gimbal 66 of Figs. 5 and 6. These similar features include base 72, which is coupled to work stand or frame 68. Four through-holes 78a-d (three of which are visible in Fig. 10), one for each pull wire, extend from c-shaped mount 76 through base 72. The pullwires feed into the through-holes 78a-d from cable housings 128 that pass through the frame 68. The more distal segments of the pullwires extend from the frame 68 into the tool cannulas 14 extending distally from the frame 68.

A ring 80 is pivotally mounted to mount 76 at pivots 82, and semi-spherical ball 84 is pivotally mounted within the ring 80 at pivots 86.

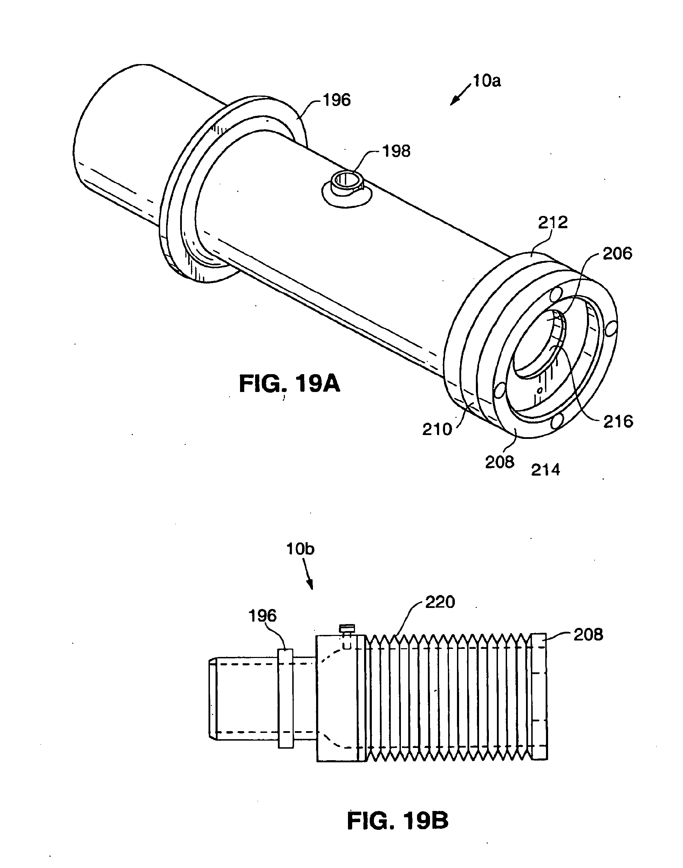

The gimbal 126 of Fig. 10 differs from the gimbal 66 of Figs. 5-6 in its use of a microadjustment assembly 130. As with the prior gimbal arrangements, the four pullwires of one of the tool cannulas terminate in the gimbal at 90 degree quadrants. Motion of the instrument shaft 18 (Fig. 1 A) alters the tension on the various pullwires, which causes deflection of the tool cannula tip and corresponding movement of the tool within the tool cannula. The effect lever arm of each pull wire is altered in the Fig. 19 embodiment by moving the point of termination of each pull wire towards or away from the gimbals' center of rotation. Moving the pullwire terminations away from the center of rotation causes movement of the tool cannula 14 to be amplified relative to the movement of the tool handle 18, whereas moving the pullwire terminations towards the center of rotation decreases the amplification.

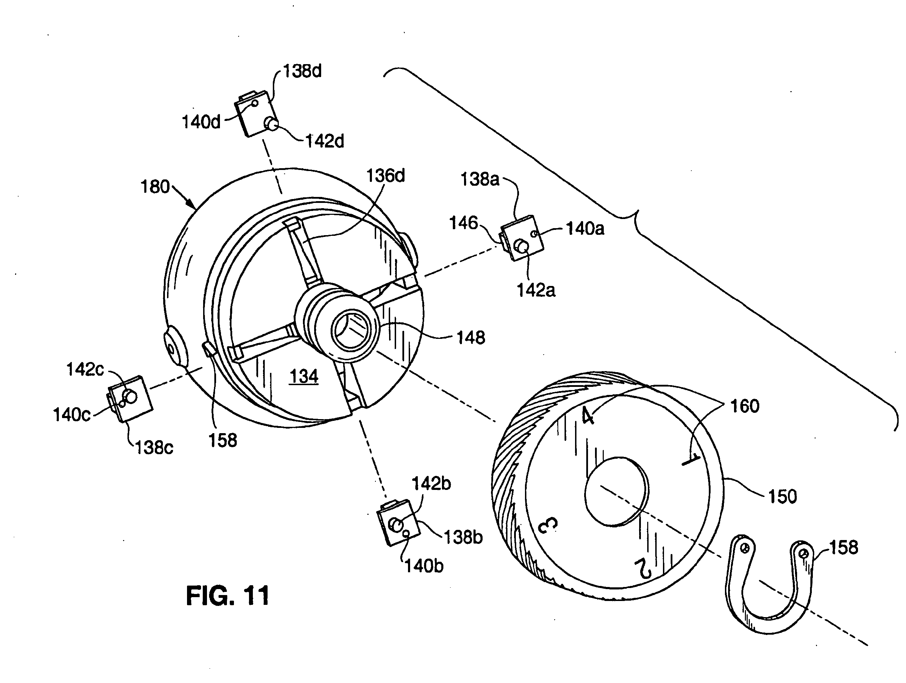

Ball 84 includes a distal surface 132 as shown in Fig. 12, and a planar proximal surface 134 as shown in Fig. 11. Four radial slots 136a-d extend through between the surfaces 132, 134. Referring to Fig. 11, four sliding terminal plates 138a-d, each including a pullwire terminal 140a-d and a proximally-extending follower pin 142a-d, are positioned in contact with the planar proximal surface 134. A peg 146 on the distal side of each terminal plate is received in the corresponding one of the slots 136a-d.

Each pullwire used to deflect the tool cannula extends through one of the slots 136a-d and is anchored within a terminal 140a-d of one of the four sliding terminals 138a-d. Fig. 12 shows the distal facing side 132 of the ball 84, with the terminals 140a-d positioned over the slots 136a-d. The pull wires themselves are not shown.

A tubular instrument port 148 is centrally positioned on the proximal surface 134 of the ball 84. A retainer cap 150 covers the surface 134, such that the instrument port 148 extends through a central opening 152 in the retainer cap. The sliding terminal plates 138a-d are sandwiched between the surface 134 and the retainer cap 150. Fig. 13 shows the cap 150 removed from the ball 84. The inner, distal facing, surface of the cap 150 includes a spiral rib 154 defining a spiral shaped slot 156. Each of the follower pins 142a-d of the terminal plates 138a-d is disposed within the spiral slot 156.

A retaining ring 158 is engaged with the instrument port 148 and functions to hold the cap 150, terminal plates 138a-d, and ball 84 together such that the follower pins 142a-d remain within the spiral slot 156. Cap is rotatable in clockwise and counterclockwise directions relative to the instrument port 148. Rotation of the cap will increase or decrease the sensitivity of the gimbal system. More specifically, if the cap is rotated in a first direction, the spiral rib 154 will cause the pins 142a-d to advance through the spiral slot towards the outer circumference of the cap, causing the terminal plates to slide radially outwardly within slots, thereby increasing the sensitivity of the gimbal system. If the cap is rotated in a second direction, the pins will advance through the spiral slot toward the center of the cap, causing the terminal plates to slide radially inwardly within the slots so as to loosen the tension on the pullwires and to decrease the sensitivity of the gimbal system. Markings 160 on the cap 150 and a corresponding pointer 158 instruct the user as to the level of sensitivity achieved when the cap is in one of the designated rotational positions relative to the pointer 158.

In alternative configurations for adjusting gimbal sensitivity, the user may have the option to set different sensitivity levels for different ones of the pull wires.

The system is preferably packed in a kit containing instructions for use instructing the user to use the system in the manner disclosed herein.

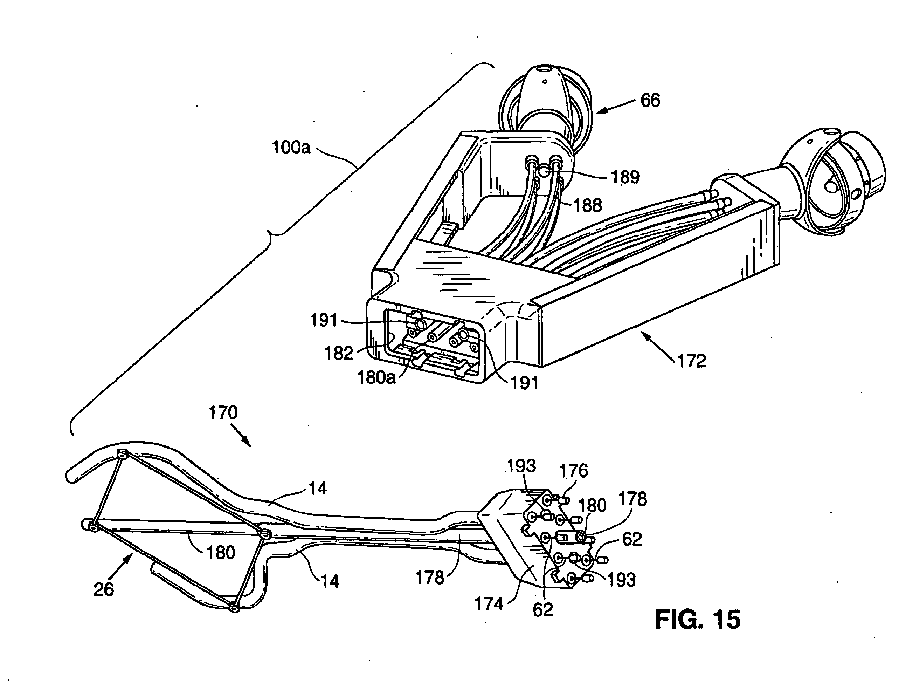

Fig. 15 shows a modified system 100A which differs from the system of Fig. 1A in that it includes a distal section 170 that is detachable from the proximal section 172 for disposal or sterilization. On the distal section 170, tool cannulas 14 extend from a hub 174, with each of the pullwires 62 from the tool cannulas 14 extending through the hub and terminating proximally of the hub as shown. Each pullwire 62 includes a head 176 or crimp on its proximal end as shown. In the Fig. 15 embodiment, a central tool cannula 178 also extends through the hub and is coupled to pivot mount 52 of the linkage 26. An additional cannula 180 (or alternatively, a tool) is coupled to the pivot mount 54 and is longitudinally moveable to deploy or collapse the linkage in a manner similar to that described in connection with Figs. 4A and 4B.

- The proximal section 172 includes a socket 182 for receiving the hub 174. A plurality of control wires 184 are positioned with their distal ends within the socket. Each control wire 184 includes a connector 186 at its distal end. Each control wire 184 extends through the frame and through a control wire tube 188. The distal end of each control wire 184 is coupled to the gimbal 126 in the same manner in which the pull wires are shown to be connected to the gimbals of Figs. 5-8B. A central port 180a (see also Fig. 9) extends through the mount 68 and allows passage of an endoscope or other tool into tool cannula 180.

During assembly of the proximal and distal sections 172, 170, the control wires 184 are coupled to corresponding ones of the tool cannula pull wires 62, so that manipulation of tool handles 18 (Fig. 1A) within the gimbals 126 will deflect the tool cannulas 14 in the same manner as described above. To connect the control wires 184 and pull wires 62, the head 176 of each pull wire 62 is inserted into and engaged with the connector 186 of a control wire 184 as illustrated in Figs. 17A and 17B. The hub 174 is seated within the socket 182 to securely connect the proximal and distal sections 172, 170.

As with the previously described embodiments, the shafts of instruments extend through instrument ports in the gimbals. See instrument 148 in the Fig. 10 embodiment. Referring again to Fig. 15, each the tool shaft (not shown but see shaft 17 in Fig. 1 A) extends through an opening 189 in the portion of mount that supports the gimbal, and extends approximately in parallel to the control wire tubes 188. The shaft further extends out a port 191 positioned in socket 182 and into a corresponding port 193 in the hub 174

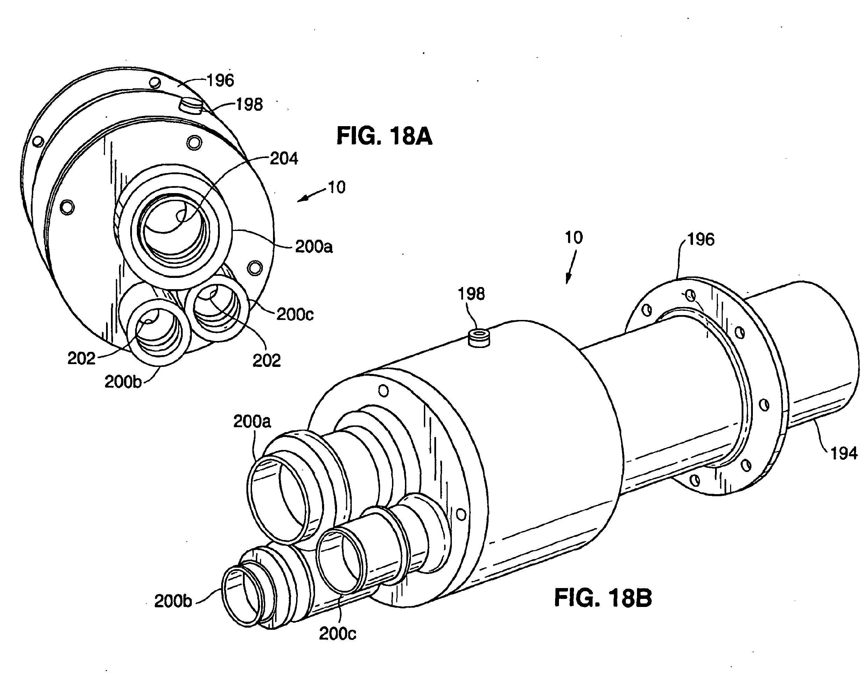

Figs. 18A and 18B give one example of a rigid access cannula 10 which includes a distal end 194 insertable into an incision formed in a body wall. The incision may be an incision or trocar puncture formed through the abdominal wall or other body wall, or through the umbilicus. The access cannula 10 may be unsupported by additional hardware, or it might include a mount that couples to a side-rail of the surgical table so as to support and stabilize the access cannula 10 throughout the procedure.

A flange 196 surrounds the external surface of the cannula 10 and is positioned to make contact with the skin surrounding the incision. A side port 198 is positioned to receive insufflation gas from an appropriate source. Insufflation gas introduced via port 198 will inflate the abdominal cavity to enlarge the working space available for the procedure. Inflation of the abdominal cavity will cause a seal to form between the flange 196 and the tissue surrounding the incision. If necessary, a substance or material (e.g. silicone, rubber, adhesive, gel, etc.) may be positioned between the flange and the tissue to facilitate sealing.

One or more flexible (e.g. rubber) fittings 200a-c extend from the proximal end of the access cannula 10. Each fitting gives access to the interior of the access cannula 10. The individual fittings 200a-c may lead to separate lumens or to a single common lumen within the access cannula. In a preferred embodiment, a single lumen having an inner diameter of 15 - 35 mm is used. During use of the system, instruments to be passed into the body are inserted through the fittings into the access cannula. As shown in Fig. 18B, seals 202 (e.g., silicone, rubber, or other suitable material) are positioned to seal against the outer surfaces of instruments such as the overtube 12 and any other instruments passed through them. Sealing is desirable to prevent loss of insufflation pressure during the procedure. Each seal has a central opening 204 that preferably has an inner diameter that is smaller than the outer diameter of the instrument or collection of instruments to be passed through it. The access cannula 10 preferably includes an internal seal that prevents loss of insufflation pressure during times when any or all of the fittings 200a-c is without an instrument. For example, if each fitting is associated with a separate lumen, duck bill valves may be positioned within each lumen to form a seal when no instrument is present in that lumen. If only a single lumen is used, a single duck bill valve may be used. Stoppers may also be positioned in the fittings when needed.

In one embodiment the access cannula 10 is approximately 15 cm (approximately 6 inches) in length.

An alternative access cannula 10a shown in Fig. 19A includes a single lumen 206 and is provided without the fittings of the Fig. 18A/18B embodiment. In the Fig. 19A embodiment, a pair of proximal, middle, and distal annular plates 208, 210, 212 are coupled to the proximal end of the cannula 10a. A proximal seal 214 is anchored-between the proximal plate 208 and the middle plate 210. A distal seal 216 is anchored between the middle plate 210 and the distal plate 212 such that the seals are spaced apart from another. The illustrated seals are annular seals each having an opening having a smaller diameter than the diameter of the overtube 12. In another variation shown in Fig. 19B, a portion of the access cannula 10b or its fittings (including one or all of the fittings of the Fig. 18A/18B embodiment) may include a longitudinally expandable bellows 220 proximal to face plate 209. Bellows 220 expand to accommodate the linkage prior to its deployment, but that can be compressed following deployment of the linkage to reduce the overall length of the access cannula 10.

The system 100 of Fig. 1A may be used for a variety of procedures to be carried out within the abdominal cavity, including resection, bypass, and/or anastomosis of the bowel, appendectomy, hysterectomy, ovary removal, cholecystectomy, prostatectomy and other procedures including those currently performed using laparoscopic or open surgical techniques. For the purpose of illustration, use of the system 100 for surgery via umbilical access will next be described with reference to the system 100 of Fig. 1A and the access cannula 10a of Fig. 19A.

The system 100 is prepared for use by feeding the distal ends of the instruments 16 into the procedural cannulas 14, with the distal ends of the instruments preferably remaining within the lumens of the procedural cannulas 14. If a central tool cannula 14a is used, the central instrument is similarly fed through that cannula 14a, and an endoscope is preferably positioned to allow visualization at the distal end of the tool cannula. The linkage 26 (which has the procedural cannulas 14 coupled to it) is placed in the collapsed position.

An incision is formed through a desired location in the abdominal wall. The umbilicus or navel may be chosen as the location for the incision since it allows access through an existing scar and avoids the necessity for additional scars. The access cannula 10a is inserted into the incision. The collapsed linkage 26/procedural cannula 14 assembly is inserted into the access cannula 10a. The proximal and distal seals 214, 216 seal against the shaft of the overtube 12.

If the cannula 10 of Figs. 18A/18B is instead used, the collapsed linkage 26/ procedural cannula 14 assembly may be inserted into the proximal end fitting 200a of the access cannula 10, an endoscope is passed into fitting 200b, and any other instrument needed for the procedure is passed into the fitting 200c. The seals in the fittings 200a-c seal against the outer surfaces of the procedural cannulas 14, endoscope, etc.

Before the linkage 26/procedural cannula 14 assembly is advanced from the access cannula 10a into the abdominal cavity, insufflation gas is introduced into the cavity via insufflation port 198 (Fig. 19A) of the access cannula 19A. Once the cavity has been inflated, the linkage 26 is moved to the expanded position as described above (e.g. by advancing central retractor 14b or procedural cannula 14a (Fig. 4A) in a distal direction using the handle 18a of central tool/cannula 14a). Expansion of the linkage 26 orients the procedural cannulas 14 as shown in Fig. 2A

The distal ends of the instruments 16 are advanced from the procedural cannulas 14, 14a and used to carry out the surgical procedure. The endoscope 20 may be advanced or oriented into a convenient position within the cavity. When reorientation of an instrument 16 is needed, the handle 18 of that instrument is manipulated, causing the associated control gimbal 126 to engage the pullwires associated with the procedural cannula 14 carrying that instrument. Once the procedure is completed, the instruments are withdrawn into the procedural cannulas 14, the linkage is collapsed (actively or by withdrawing it into the access cannula 10a). Any other instruments similarly withdrawn from the access cannula, the access cannula 10 is removed from the body, and the incision is closed in the usual fashion.

Fig. 22 schematically illustrates use of the disclosed system of Fig. 2 as used such as for a cholecystectomy procedure. According to such a procedure, the overtube 12 (with the procedural cannulas 14 extending through it) is introduced into the peritoneal space via a single abdominal port (not shown) and oriented towards the procedural site as shown. The overtube may be straight, but it will preferably have a bend tailored towards the quadrant of the abdominal cavity within which the procedure is to be carried out. Differently shaped overtubes may be used for different approaches (e.g. upper right quadrant vs. upper left quadrant approaches). The liver retractor 16c or retractor 16a (Fig. 2A) is used to lift and retract the liver superiorly away from the gallbladder and the operational area of the instruments 16. Instruments 16 are advanced through the procedural cannulas and used to perform the procedure. Whereas prior art laparoscopic procedures involve formation of three surgical ports or incisions labelled W (retractor port), X (right tool port), Y (scope port), Z (left tool port) in Fig. 22, use of the disclosed system allows the cholecystectomy procedure to be performed less invasively while allowing the surgeon to carry out the procedure from the same familiar perspective from which s/he would have performed the laparoscopic procedure. Using the linkage system tools in the tool cannulas, the central retractor, and the scope are oriented to approach the operative site from the approximate directions that they would have taken if they had been advanced through ports X, Y, W and Z.

The illustrated embodiments utilize internal scaffold devices in single port procedures to locate tools at or near the abdominal walls such that the tools may be manipulated in a way that is intuitive to the surgeon given his/her experience with laparoscopic and/or open surgical techniques,

While certain embodiments have been described above, it should be understood that these embodiments are presented by way of example, and not limitation.

## Claims

1. A single port surgical system (100, 100A) comprising:
first and second tool cannulas (14), each tool cannula having a lumen for receiving a tool for performing a procedure within a body cavity;
a longitudinal tool cannula (14a, 14c) disposed between the first and second tool cannulas;
a two-member linkage (26, 26a) for adjusting relative positions of the first and second tool cannulas within the body cavity, the two-member linkage having:
a first member (28) having a first end pivotally coupled to the first tool cannula and a second end pivotally coupled to the longitudinal tool cannula; and
a second member (28) having a first end pivotally coupled to the second tool cannula and
a second end pivotally coupled to the longitudinal tool cannula;
wherein the longitudinal tool cannula is longitudinally slidable relative to the first and second tool cannulas between first and second positions, wherein movement of the longitudinal tool cannula from the first position to the second position pivots the first and second members laterally outward relative to the longitudinal tool cannula, thereby increasing a distance between the first and second tool cannulas.

2. The system of claim 1, wherein the system includes an overtube (12), and wherein the first, second and longitudinal tool cannulas extend through the overtube.

3. The system of claim 2, wherein the first and second tool cannulas are fixed within the overtube.

4. The system of claim 2, further including an endoscope (20) extending through the overtube.

5. The system of claim 1, further including at least one actuator operatively associated with the first tool cannula such that manipulation of the actuator deflects a distal portion of the first tool cannula.

6. The system of claim 5, wherein the first tool cannula includes a plurality of pull wires (62) coupled to its distal portion, the distal portion deflectable in response to application of tension on at least one of the pull wires.

7. The system of claim 6, wherein actuator comprises a gimbal (66, 126) and wherein the pull wires (62) include proximal ends coupled to the gimbal, the gimbal moveable in multiple directions to apply tension to the pull wires.

8. The system of claim 7, wherein the gimbal includes a tool port having an opening, wherein the gimbal is moveable by movement of the tool port, and wherein the tool port is proportioned to receive a distal end of a tool (16) as the distal end of the tool is advanced into the tool cannula.

9. The system of claim 8, wherein the tool port is moveable in response to movement of a handle (18) of a tool positioned within the tool port.

10. The system of claim 1, wherein the longitudinal tool cannula in the second position positions the first and second tool cannulas such that a lateral distance between the first and second tool cannulas is 7.5 cm (3 inches) or more.

11. The system of claim 1, wherein the longitudinal tool cannula in the second position positions the first and second tool cannulas such that a lateral distance between the first and second tool cannulas is 10 cm (4 inches) or more.

12. The system of claim 5, wherein the system further includes a mount (68), and a pair of actuators on the mount.

13. The system of claim 12, wherein each actuator includes an instrument port (90), such that a tool positioned in one of the first and second tool cannulas extends through a corresponding one of the instruments ports, and wherein movement of a handle of a tool within the instrument port actuates the corresponding actuator to deflect a tool cannula.

14. The system of claim 12, wherein the mount is attachable to a fixture in an operating room.

15. The system of claim 14, wherein the fixture is selected from the collection of fixtures consisting of surgical tables, ceiling fixtures, and carts.

16. The system of claim 13, wherein each of the first and second tool cannulas includes a plurality of pull wires coupled to a distal portion of the corresponding tool cannula, wherein the pullwires of each tool cannula are coupled to one of the actuators, and wherein movement of the tool handles within the instrument ports activates the corresponding pullwires.

17. The system of claim 12, wherein the mount includes a distal portion (170) and a proximal portion (172) detachable from the distal portion, wherein the tool cannulas are mounted on the distal portion and wherein the actuators are on the proximal portion.

## Patentansprüche

1. Chirurgisches System (100, 100A) mit einem einzigen Zugang, das Folgendes umfasst:
eine erste und eine zweite Werkzeugkanüle (14), wobei jede Werkzeugkanüle ein Lumen zum Aufnehmen eines Werkzeugs zum Ausführen einer Prozedur in einer Körperhöhle hat;
eine longitudinale Werkzeugkanüle (14a, 14c), die zwischen der ersten und der zweiten Werkzeugkanüle angeordnet ist;
ein Zwei-Elemente-Gestänge (26, 26a) zum Einstellen von relativen Positionen der ersten und der zweiten Werkzeugkanüle in der Körperhöhle, wobei das Zwei-Elemente-Gestänge Folgendes beinhaltet:
ein erstes Element (28) mit einem mit der ersten Werkzeugkanüle schwenkbar gekoppelten ersten Ende und einem mit der longitudinalen Werkzeugkanüle schwenkbar gekoppelten zweiten Ende; und
ein zweites Element (28) mit einem mit der zweiten Werkzeugkanüle schwenkbar gekoppelten ersten Ende und einem mit der longitudinalen Werkzeugkanüle schwenkbar gekoppelten zweiten Ende;
wobei die longitudinale Werkzeugkanüle longitudinal relativ zur ersten und zur zweiten Werkzeugkanüle zwischen der ersten und der zweiten Position verschiebbar ist, wobei durch die Bewegung der longitudinalen Werkzeugkanüle von der ersten Position in die zweite Position das erste und zweite Element lateral nach außen relativ zu der longitudinalen Werkzeugkanüle geschwenkt werden, um dadurch einen Abstand zwischen der ersten und der zweiten Werkzeugkanüle zu vergrößern.

2. System nach Anspruch 1, wobei das System einen Übertubus (12) beinhaltet und wobei die erste, die zweite und die longitudinale Werkzeugkanüle durch den Übertubus verlaufen.

3. System nach Anspruch 2, wobei die erste und die zweite Werkzeugkanüle in dem Übertubus fixiert sind.

4. System nach Anspruch 2, das ferner ein durch den Übertubus verlaufendes Endoskop (20) aufweist.

5. System nach Anspruch 1, das ferner wenigstens einen Aktuator beinhaltet, der mit der ersten Werkzeugkanüle operativ assoziiert ist, so dass eine Manipulation des Aktuators einen distalen Teil der ersten Werkzeugkanüle ablenkt.

6. System nach Anspruch 5, wobei die erste Werkzeugkanüle mehrere mit ihrem distalen Teil gekoppelte Zugdrähte (62) aufweist, wobei der distale Teil als Reaktion auf das Beaufschlagen wenigstens eines der Zugdrähte mit Zugspannung ablenkbar ist.

7. System nach Anspruch 6, wobei der Aktuator einen Kardanring (66, 126) umfasst und wobei die Zugdrähte (62) mit dem Kardanring gekoppelte proximale Enden aufweisen, wobei der Kardanring in mehreren Richtungen beweglich ist, um die Zugdrähte mit Zugspannung zu beaufschlagen.

8. System nach Anspruch 7, wobei der Kardanring einen Werkzeugzugang mit einer Öffnung aufweist, wobei der Kardanring durch Bewegen des Werkzeugzugangs beweglich ist und wobei der Werkzeugzugang so proportioniert ist, dass er ein distales Ende eines Werkzeugs (16) aufnimmt, während das distale Ende des Werkzeugs in die Werkzeugkanüle vorgeschoben wird.

9. System nach Anspruch 8, wobei der Werkzeugzugang als Reaktion auf eine Bewegung eines Griffs (18) eines in dem Werkzeugzugang positionierten Werkzeugs beweglich ist.

10. System nach Anspruch 1, wobei die longitudinale Werkzeugkanüle in der zweiten Position die erste und die zweite Werkzeugkanüle so positioniert, dass ein lateraler Abstand zwischen der ersten und der zweiten Werkzeugkanüle 7,5 cm (3 Zoll) oder mehr beträgt.

11. System nach Anspruch 1, wobei die longitudinale Werkzeugkanüle in der zweiten Position die erste und die zweite Werkzeugkanüle so positioniert, dass ein lateraler Abstand zwischen der ersten und der zweiten Werkzeugkanüle 10 cm (4 Zoll) oder mehr beträgt.

12. System nach Anspruch 5, wobei das System ferner eine Halterung (68) und ein Paar Aktuatoren an der Halterung aufweist.

13. System nach Anspruch 12, wobei jeder Aktuator einen Instrumentenzugang (90) aufweist, so dass ein in der ersten oder der zweiten Werkzeugkanüle positioniertes Werkzeug durch einen entsprechenden einen der Instrumentenzugänge verläuft, und wobei eine Bewegung eines Griffs eines Werkzeugs in dem Instrumentenzugang den entsprechenden Aktuator betätigt, um eine Werkzeugkanüle abzulenken.

14. System nach Anspruch 12, wobei die Halterung an einer Haltevorrichtung in einem Operationssaal angebracht werden kann.

15. System nach Anspruch 14, wobei die Haltevorrichtung aus der Sammlung von Haltevorrichtungen bestehend aus Operationstischen, Deckenhaltevorrichtungen und Wagen ausgewählt ist.

16. System nach Anspruch 13, wobei die erste und zweite Werkzeugkanüle jeweils mehrere mit einem distalen Teil der entsprechenden Werkzeugkanüle gekoppelte Zugdrähte aufweisen, wobei die Zugdrähte jeder Werkzeugkanüle mit einem der Aktuatoren gekoppelt sind und wobei durch eine Bewegung der Werkzeuggriffe in den Instrumentenzugängen die entsprechenden Zugdrähte aktiviert werden.

17. System nach Anspruch 12, wobei die Halterung einen distalen Teil (170) und einen vom distalen Teil abnehmbaren proximalen Teil (172) aufweist, wobei die Werkzeugkanülen am distalen Teil montiert sind und wobei die Aktuatoren sich am proximalen Teil befinden.

## Revendications

1. Système chirurgical à orifice unique (100, 100A) comprenant :
une première et une deuxième canule pour instrument (14), chaque canule pour instrument ayant un lumen pour recevoir un instrument destiné à effectuer une procédure dans une cavité corporelle ;
une canule longitudinale pour instrument (14a, 14c) disposée entre la première et la deuxième canule ;
une tringlerie à deux membres (26, 26a) pour ajuster des positions relatives de la première et de la deuxième canule pour instrument à l'intérieur de la cavité corporelle, la tringlerie à deux membres ayant :
un premier membre (28) ayant une première extrémité accouplée d'une manière pivotante à la première canule pour instrument et un deuxième membre accouplé d'une manière pivotante à la canule longitudinale pour instrument; et
un deuxième membre (28) ayant une première extrémité accouplée d'une manière pivotante à la deuxième canule pour instrument et une deuxième extrémité accouplée d'une manière pivotante à la canule longitudinale pour instrument;
dans lequel la canule longitudinale pour instrument peut coulisser dans le plan longitudinal par rapport à la première et à la deuxième canule pour instrument entre une première et une deuxième position, où le déplacement de la canule longitudinale pour instrument de la première position à la deuxième position fait pivoter le premier et le deuxième membre latéralement vers l'extérieur par rapport à la canule longitudinale pour instrument, accroissant ainsi une distance entre la première et la deuxième canule pour instrument.

2. Système selon la revendication 1, où le système comprend un tube de couverture (12) et dans lequel la première, la deuxième et la canule longitudinale pour instrument s'étendent à travers le tube de couverture.

3. Système selon la revendication 2, dans lequel la première et la deuxième canule sont fixes dans le tube de couverture.

4. Système selon la revendication 2, comprenant en outre un endoscope (20) s'étendant à travers le tube de couverture.

5. Système selon la revendication 1, comprenant en outre au moins un actionneur associé d'une manière opérationnelle à la première canule pour instrument de telle sorte qu'une manipulation de l'actionneur défléchit une partie distale de la première canule pour instrument.

6. Système selon la revendication 5, dans lequel la première canule pour instrument comprend une pluralité de fils de tirage (62) accouplés à sa partie distale, la partie distale pouvant être défléchie en réponse à l'application d'une tension sur au moins l'un des fils de tirage.

7. Système selon la revendication 6, dans lequel l'actionneur comprend un cardan (66, 126) et dans lequel les fils de tirage (62) comprennent des extrémités proximales accouplées au cardan, le cardan étant déplaçable dans des directions multiples afin d'appliquer une tension aux fils de tirage.

8. Système selon la revendication 7, dans lequel le cardan comprend un orifice pour instrument ayant une ouverture, dans lequel le cardan est déplaçable par le déplacement de l'orifice pour instrument et dans lequel l'orifice pour instrument est proportionné pour recevoir une extrémité distale d'un instrument (16) tandis que l'extrémité distale de l'instrument est avancée dans la canule pour instrument.

9. Système selon la revendication 8, dans lequel l'orifice pour instrument est déplaçable en réponse au déplacement d'une poignée (18) d'un instrument positionné dans l'orifice pour instrument.

10. Système selon la revendication 1, dans lequel la canule longitudinale pour instrument dans la deuxième position positionne la première et la deuxième canule pour instrument de telle sorte qu'une distance latérale entre la première et la deuxième canule pour instrument est de 7,5 cm (3 pouces) ou plus.

11. Système selon la revendication 1, dans lequel la canule longitudinale pour instrument dans la deuxième position positionne la première et la deuxième canule pour instrument de telle sorte qu'une distance latérale entre la première et la deuxième canule pour instrument est de 10 cm (4 pouces) ou plus.

12. Système selon la revendication 5, où le système comprend en outre une monture (68), et une paire d'actionneurs sur la monture.

13. Système selon la revendication 12, dans lequel chaque actionneur comprend un orifice pour instrument (90), de telle sorte qu'un instrument positionné dans l'une d'entre la première et la deuxième canule pour instrument s'étend à travers un orifice correspondant des orifices pour instrument, et dans lequel le déplacement d'une poignée d'un instrument dans l'orifice pour instrument actionne un actionneur correspondant pour défléchir une canule pour instrument.

14. Système selon la revendication 12, dans lequel la monture peut être attachée à une pièce fixe dans une salle opératoire.

15. Système selon la revendication 14, dans lequel la pièce fixe est sélectionnée parmi la collection de pièces fixes consistant en tables d'opération, pièces fixes au plafond et chariots.

16. Système selon la revendication 13, dans lequel chacune de la première et de la deuxième canule pour instrument comprend une pluralité de fils de tirage accouplés à une partie distale de la canule pour instrument correspondante, dans lequel les fils de tirage de chaque canule pour instrument sont accouplés à l'un des actionneurs, et dans lequel le déplacement des poignées d'instrument dans les orifices pour instruments actionne les fils de tirage correspondants.

17. Système selon la revendication 12, dans lequel la monture comprend une partie distale (170) et une partie proximale (172) détachable de la partie distale, dans lequel les canules pour instrument sont montées sur la partie distale et dans lequel les actionneurs sont sur la partie proximale.
